# FASCICULE DE BREVET EUROPEEN

(11) **EP 1 217 073 B1**
(45) Date de publication et mention de la délivrance du brevet: **15.03.2006**
(21) Numéro de dépôt: 01130564.6
(22) Date de dépôt: 18.07.1996
(51) Int. Cl.: C12N 15/54, C12N 15/82, A01H 5/00

(54) **Plantes transformées à tolérance accrue aux herbicides de la famille des phosphométhylglycines contenant un gène codant pour une 5-énol pyruvylshikimate-3- phosphate synthase mutée**
Transgene Pflanzen mit verbesserter Toleranz gegen Herbizide der Phosphomethylglycinfamilie, die ein für mutierte 5-enolpyruvylshikimate-3-phosphate Synthase kodierendes Gen enthalten
Transformed plants with improved tolerance to phosphomethylglycine family herbicides containing a gene encoding a mutated 5-enolpyruvylshikimate-3-phophate synthase

(30) Priorité: 19.07.1995 FR 9508979
(43) Date de publication de la demande: 26.06.2002
(62) Demande divisionnaire de: 96925812.8
(73) Titulaire: Bayer CropScience S.A., 69009 Lyon (FR)
(72) Inventeur: Lebrun, Michel, 34090 Montpellier (FR); Sailland, Alain, 69370 Saint Didier au Mont d'or (FR); Freyssinet, Georges, 69450 Saint Cyr Au Mont d'Or (FR)

(56) Documents cités:
- EP-A- 0 293 358
- EP-A- 0 507 698
- EP-A- 0 508 909
- EP-A- 0 633 317
- EP-A- 0 652 286
- WO-A-91/04323
- WO-A-91/09948
- WO-A-92/06201
- WO-A-93/06713
- WO-A-93/19189
- WO-A-95/06128
- WO-A-97/04114
- WO-A-98/02562
- RUFF T., ET AL.: "EFFECTS OF AMINO ACID SUBSTITUTIONS ON GLYPHOSATE TOLERANCE AND ACTIVITY OF EPSPS. ALBUQUERQUE, NEW MEXICO, USA, JULY 28-AUGUST 1, 1991. PLANT PHYSIOL(BETHESDA) 96 (1 SUPPL.). 1991. 94." ANNUAL MEETING OF THE AMERICAN SOCIETY OF PLANT PHYSIOLOGISTS,, XP002023052
- PADGETTE S R, ET AL.: "SITE-DIRECTED MUTAGENESIS OF A CONSERVED REGION OF THE 5 ENOLPYRUVYLSHIKIMATE-3-PHOSPHATE SYNTHASE ACTIVE SITE." J BIOL CHEM 266 (33). 1991. 22364-22369., XP002023053

## Description

La présente invention concerne une nouvelle 5-enol pyruvylshikimate-3-phosphate synthase (ou EPSPS), qui présente une tolérance accrue vis à vis des herbicides inhibiteurs compétitifs vis à vis du phosphonoenolpyruvate (PEP) de l'activité EPSPS. Cette EPSP synthase plus tolérante présente au moins une substitution "Thréonine par Isoleucine". Elle concerne également un gène codant pour une telle protéine, des cellules végétales transformées par des constructions gènes chimères contenant ce gène, les plantes régénérées à partir de ces cellules ainsi que les plantes issues de croisement utilisant ces plantes tranformées.

Le glyphosate, le sulfosate ou la fosamétine sont des herbicides systémiques à large spectre de la famille des phosphonométhylglycines. Ils agissent essentiellement comme inhibiteurs compétitifs de la 5-enol pyruvylshikimate-3-phosphate synthase (EC 2.5.1.19) ou EPSPS vis à vis du PEP(phosphoenolpyruvate). Après leur application sur la plante, ils sont véhiculés dans la plante où ils s'accumulent dans les parties à croissance rapide, notamment les apex caulinaires et racinaires, provoquant l'altération jusqu'à la destruction des plantes sensibles.

L'EPSPS plastidiale, cible principale de ces produits est une enzyme de la voie de biosynthèse des acides aminés aromatiques, qui est codée par un ou des gènes nucléaires et synthétisée sous forme d'un précurseur cytoplasmique puis importée dans les plastes où elle s'accumule sous sa forme mature.

La tolérance des plantes au glyphosate et aux produits de la famille est obtenue par introduction stable dans leur génome d'un gène d'EPSPS d'origine végétale ou bactérienne mutée ou non quant aux caractéristiques d'inhibition par le glyphosate du produit de ce gène. Etant donné le mode d'action du glyphosate et le degré de tolérance au glyphosate du produit des gènes utlisés, il est intéressant de pouvoir exprimer le produit de la traduction de ce gène de façon à permettre son accumulation importante dans les plastes.

D est connu, par exemple d'après le brevet américain 4 535 060, de conférer à une plante une tolérance à un herbicide du type ci-dessus, en particulier la N-phosphonométhylglycine ou glyphosate, par introduction dans le génome des plantes d'un gène codant pour une EPSPS portant au moins une mutation rendant cette enzyme plus résistante à son inhibiteur compétitif (le glyphosate) après localisation de l'enzyme dans le compartiment plastidial. Ces techniques demandent cependant à être améliorées pour obtenir une plus grande fiabilité dans l'emploi de ces plantes en conditions agronomiques.

Des EPSPS de plantes mutées et leur utilisation pour la préparation de plantes à tolérance améliorée au glyphosate sont mentionnées dans les demandes de brevet EP 293538, WO 91/04323 et WO 92/06201. Des EPSPS dites de Classe Il sont identifiées dans la demande de brevet WO 92/04449. D'autres gènes de tolérance au glyphosate sont mentionnés dans la demande de brevet WO 95/06128.

Dans la présente description, on entend par "plante" tout organisme multicellulaire différencié capable de photosynthèse et par "cellule végétale" toute cellule issue d'une plante et pouvant constituer des tissus indifférenciés tels que des cals ou des tissus différenciés tels que des embryons ou des parties de plantes ou des semences.

La présente invention a pour objet la production de plantes transformées ayant une tolérance accrue aux herbicides de la famille des phosphonométhylglycines par régénération de cellules transformées à l'aide de nouveaux gènes chimères comportant un gène de tolérance à ces herbicides.

La présente invention concerne un gène ADN codant pour une 5-enol pyruvylshikimate-3-phosphate synthase (EPSPS) mutée, caractérisé en ce qu'il comprend au moins une substitution Thréonine 102 → Isoleucine et une mutation constituée par une substitution Proline 106 par la Sérine.

Le gène selon l'invention est de préférence d'origine végétale, en particulier d'origine de maïs.

L'invention concerne un gène chimérique comprenant une séquence codante ainsi que des éléments de régulation en position 5' et 3'hétérologues pouvant fonctionner dans les plantes, caractérisé en ce qu'il comprend, comme séquence codante, au moins une séquence d'un gène décrit précédemment. Le gène chimérique comprend un promoteur de virus de plante ou un promoteur de plante (ex: tubuline, histone, introns, actine...).

L'invention a également pour objet un gène chimère pour conférer aux plantes une tolérance accrue vis à vis d'un herbicide ayant pour cible l'EPSPS, comprenant, dans le sens de la transcription: une zone promotrice, éventuellement une zone peptide de transit, une séquence d'un gène codant pour une enzyme de tolérance au glyphosate et une zone signal de polyadénylation non traduite en 3', caractérisé en ce que le gène de tolérance au glyphosate comporte, par rapport au gène dont il est dérivé, une substitution "Thréonine 102 par Isoleucine" dans la zone "aroA"(EPSPS). De manière préférée, elle comprend en outre, dans la même zone une substitution "Proline 106 par Sérine". Ces substitutions peuvent être introduites, ou être présentes, dans une séquence d'EPSPS d'origine quelconque: notamment végétale, bactérienne, d'algues ou de champignon.

Les peptides de transit utilisables dans la zone de peptide de transit peuvent être en soi connus, d'origine végétale, par exemple issus de maïs, de tournesol, de pois, de tabac ou autres. Le premier et le second peptide de transit peuvent identiques, analogues ou différents. Ils peuvent en outre comprendre chacun une ou plusieurs unités peptide de transit selon la demande de brevet européen EP 0 508 909. Cette zone caractéristique a comme rôle de permettre le relargage d'une protéine mature et native, et en particulier l'EPSPS mutée ci-dessus, avec une efficacité maximale dans le compartiment plasmidique.

La zone promotrice du gène chimère selon l'invention peut être composée avantageusement d'au moins un promoteur ou un fragment d'un promoteur de gène s'exprimant naturellement dans les plantes...(tubuline, introns actine, histone).

La zone signal de terminaison de la transcription non traduite en 3' du gène chimère peut être d'origine quelconque, par exemple bactérienne telle que celle du gène de la nopaline synthase, ou végétale telle que celle du gène histone H4A748 d' *Arabidopsis thaliana* selon la demande de brevet européen (demande européenne 633 317).

Le gène chimère selon l'invention peut comprendre, en plus des parties essentielles ci-dessus, au moins une zone intermédiaire non traduite (linker), qui peut être localisés entre les différentes régions transcrites décrites ci-dessus. Cette zone intermédiaire peut être d'origine quelconque, par exemple bactérienne, virale ou végétale.

L'invention concerne aussi un vecteur pour la transformation des plantes, caractérisé en ce qu'il comprend, au moins un gène chimérique tel que défini précédemment.

Elle concerne également une cellule végétale, caractérisée en ce qu'elle comprend au moins un gène chimère tel que défini précédemment.

La présente invention concerne enfin un procédé de traitement des plantes avec un herbicide ayant l'EPSPS pour cible, caractérisé en ce que l'on applique l'herbicide à des 30 plantes transformées selon l'invention. L'herbicide est en particulier du glyphosate ou un précurseur du glyphosate.

Isolement d'un ADNc codant pour une EPSPS de maïs:

Les différentes étapes, qui ont conduit à l'obtention de l'ADNc d'EPSPS de maïs, qui a servi de substrat à l'introduction des deux mutations, sont décrites ci-dessous. Toutes les opérations décrites ci-dessous sont données à titre d'exemples et correspondent à un choix, effectué parmi les différentes méthodes disponibles pour parvenir au même résultat. Ce choix n'a aucune incidence sur la qualité du résultat et par conséquent, toute méthode adaptée peut être utilisée par l'homme de l'art pour parvenir au même résultat. La plupart des méthodes d'ingénierie des fragments d'ADN sont décrites dans "Current Protocols in Molecular Biology" Volumes 1 et 2, Ausubel F.M. et al , publiés par Greene Publishing Associates et Wiley -Interscience (1989)(Par la suite, les références à des protocoles décrits dans cet ouvrage seront notées "réf. CPMB"). Les opérations concernant l'ADN, qui ont été effectuées selon les protocoles décrits dans cet ouvrage sont, en particulier les suivantes: ligation de fragments d'ADN, traitements par l'ADN polymérase de Klénow et la T4 ADN polymérase, préparation d'ADN de plasmides et de bactériophages λ soit en minipréparation soit en maxi préparation, analyses d'ADN et d'ARN respectivement selon les techniques de Southern et Northern. D'autres méthodes décrites dans cet ouvrage ont été suivies, et seules les modifications ou ajouts significatifs à ces protocoles ont été décrits ci-dessous.

### Exemple 1:

1. Obtention d'un fragment d'EPSPS d'*Arabidopsis thaliana*
   a) deux oligonucleotides 20-mers de séquences respectives: ont été synthétisés à partir de la séquence d'un gène d'EPSPS d'*Arabidopsis thaliana* (Klee H.J. et al. (1987) Mol. Gen. Genet., 210, 437-442). Ces deux oligonucleotides sont respectivement en position 1523 à 1543 et 1737 à 1717 de la séquence publiée et en orientation convergente.
   b) L'ADN total d'*Arabidopsis thaliana* (*var*. *columbia*) a été obtenu chez Clontech (référence catalogue: 6970-1)
   c) On mélange 50 nanogrammes(ng) d'ADN avec 300ng de chacun des oligonucleotides et soumis à 35 cycles d'amplification avec un appareil Perkin-Elmer 9600, dans les conditions de milieu standard pour l'amplification préconisées par le fournisseur. Le fragment de 204pb résultant constitue le fragment d'EPSPS d' *Arabidopsis thaliana.*
2. Construction d'une bibliothèque d'un ADNc à partir d'une ligne cellulaire de maïs BMS.
   a) On broye 5 g de cellules filtrées dans l'azote liquide et les acides nucléiques totaux extraits selon la méthode décrite par Shure et al. avec les modifications suivantes:
      - le pH du tampon de lyse est ajusté à PH = 9,0;
      - après la précipitation par l'isopropanol, le culot est repris dans l'eau et après dissolution, ajusté à 2,5 M LiCl. Après incubation pendant 12 h à °C, le culot de la centrifugation d 15 min. à 30000g à 4°C est resolubilisé. L'étape de précipitation par LiCl est alors répétée. Le culot resolublisé constitue la fraction ARN des acides nucléiques totaux.
   b) La fraction ARN-polyA+ de la fraction ARN est obtenue par chromatographie sur colonne oligo-dT cellulose telle que décrite dans "Current Protocols in Molecular Biology".
   c) Synthèse d'ADNc double brin à extrémité synthétique EcoRI: elle est réalisée en suivant le protocole du fournisseur des différents réactifs nécessaires à cette synthèse sou forme d'un kit:le "copy kit" de la société In Vitrogen.
      Deux oligonucleotides simples brins et partiellement complémentaires de séquences respectives: sont ligués avec les ADNc double brin à extrémités franches.
      Cette ligation des adaptateurs résulte en la création de sites Sma I accolés aux ADNc double brin et EcoRI sous forme cohésive à chaque extrélité des ADNC double brin.
   d) Création de la bibliothèque:
      Les ADNc présentant à leurs extrémités les sites artificiels cohésifs EcoRI sont ligués avec le ADNc du bactériophage λgt10 coupé par EcoRI et déphosphorylé selon le protocole du fournisseur Naew England Biolabs.
      Une aliquote de la réaction de ligation a été encapsidée *in vitro* avec des extraits d'encapsidation: Gigapack Gold selon les instructions du fournisseur, cette librairie a été titrée en utilisant la bactérie *E.coli* C600*hfl*. la librairie ainsi obtenue est amplifiée et stockée selon les instructions du même fournisseur et constitue la librairie de ADNc de suspension cellulaire de maïs BMS.
3. Criblage de la bibliothèque de ADNc de suspension cellulaire de mais BMS avec la sonde EPSP d'*Arabidopsis thaliana:*
   Le protocole suivi est celui de "Current Protocols in Molecular Biology" Volumes 1 et 2, Ausubel F.M. et al, publiés par Greene Publishing Associates et Wiley-Interscience (1989)(CPMB). En bref, environ 10⁶ phages recombinants sont étalés sur boîte LB à une densité moyenne de 100 phages /cm². Les plages de lyses sont répliqués en doubles sur membrane Hybond N d'Amersham.
   h) L'ADN a été fixé sur les filtres par traitement UV 1600kJ (Stratalinker de Stratagene). Les filtres iont été préhybridés dans: 6xSSC/0,1%SDS/0,25 lait écrémé pendant 2h à 65°C. La sonde EPSPS *d'Arabidopsis thaliana* a été marquée au ³²P-dCTP par "random-priming" selon les instructions du fournisseur (Kit Ready to Go de Pharmacia). L'activité spécifique obtenue est de l'order de 10⁸ cpm par µg de fragment. Après dénaturation pendant 5 min à 100°C, la sonde est ajoutée dans le milieu de préhybridation et l'hybridation est poursuivie pendant 14 heures à 55°C. Les filtres sont fluorographiés 48h à -80°C avec un film KodakXAR5 et des écrans renforçateurs Hyperscreen RPN d'Amersham. L'alignement des spots positifs sur le filtre avec les boîtes d'où ils sont issus permet de prélever, sur la boîte, des zones correspondant aux phages présentant une réponse d'hybridation positive avec la sonde EPSPS *d'Arabidopsis thaliana.* Cette étape d'étalement, transfert, hybridation, récupération est répétée jusqu'à ce que tous les spots de la boîte des phages successivement purifiés se révèlent positifs à 100% en hybridation. Une plage de lyse par phage indépendant est alors prélevée dans du milieu λ diluant (Tris-CI pH= 7,5; MgSO4 10mM; NaCl 0,1M; gélatine 0,1%), ces phages en solution constituant les clones positifs de 1' EPSP de la suspension cellulaire de maïs BMS.
4. Préparation et analyse de l'ADN des clones d'EPSP de la suspension cellulaire de maïs BMS.
   On ajoute environ 5.10⁸ phages à 20 ml de bactéries C600hfl à 2 OD 600nm/ml et incubés 15 minutes à 37°C. Cette suspension est alors diluée dans 200ml de milieu de croissance des bactéries dans un Erlen de 11 et agitée dans un agitateur rotatif à 250 rpm. La lyse est constatée par clarification du milieu, correspondant à 1 lyse des bactéries turbides et se produit après environ 4 h d'agitation. Ce surnageant est alors traité comme décrit dans "Current Protocols in Molecular Biology". L'ADN obtenu correspond aux clones d'EPSP de la suspension cellulaire de mais BMS.
   Un à deux µg de cet ADN sont coupés par EcoRI et séparés sur gel d'agarose LGTA/TBE (réf. CPMB) à 0,8%. Une dernière vérification consiste à s'assurer que l'ADN purifié présente bien un signal d'hybridation avec la sonde EPSPS d'*Arabidopsis thaliana.* Après l'électrophorèse, les fragments d'ADN sont transférés sur membrane Hybond N d'Amersham selon le protocole de Southern décrit dans "Current Protocols in Molecular Biology". Le filtre est hybridé avec la sonde EPSPS d'*Arabidopsis thaliana* selon les conditions décrites au paragraphe 3 ci-dessus. Le clone présentant un signal d'hybridation avec la sonde EPSPS d'*Arabidopsis thaliana* et contenant le plus long fragment EcoRI a une taille estimée sur gel à environ 1,7kpb.
5. Obtention du clone pRPA-ML-711:
   Dix µg de l'ADN du clone phagique contenant l'insert de 1,7kpb sont digérés par EcoRI et séparés sur gel d'agarose LGTA/TBE (réf. CPMB) à 0,8%. Le fragment de gel contenant l'insert de 1,7kpb est excisé du gel par coloration BET et le fragment est traité à la β-agarse selon le protocole du fournisseur New England Biolabs. L'ADN purifié du fragment de 1,7kpb est ligué à 12°C pendant 14h avec l'ADN du plasmide pUC 19 (New England Biolabs) coupé par EcoRI selon le protocole de ligation décrit dans "Current Protocols in Molecular Biology". Deux µl du mélange de ligation ci-dessus sont utilisés pour la transformation d'une aliquote d'E.coli DH10B électro compétentes ; la transformation se fait par électroporation en utilisant les conditions suivantes: le mélange de bactéries compétentes et et de milieu de ligation est introduit dans une cuvette d'électroporation d'épaisseur 0,2cm (Biorad) prélablement refroidie à 0°C. Les conditions physiques de l'électroporation utilisant un électroporateur de marque Biorad sont 2500 Volts, 25 µFarad et 200 Ω. Dans ces conditions, le temps de décharge moyen de condensateur est de l'ordre de 4,2 millisecondes. Les bactéries sont alors reprises dans 1 ml de milieu SOC (réf. CPMB) et agitées pendant 1 heure à 200 rpm sur un agitateur rotatif dans des tubes Coming de 15 ml. Après étalement sur milieu LB/agar supplémenté à 100 µg/ml de carbéniciline, les mini-préparations des clones bactériens ayant poussé après une nuit à 37 °C est réalisée selon le protocole décrit dans "Current Protocols in Molecular Biology". Après digestion par EcoRI de l'ADN et séparation en électrophorèse sur gel d'agarose LGTA/TBE (réf. CPMB) à 0,8%, les clones présentant un insert de 1,7kpb sont conservés. Une dernière vérification consiste à s'assurer que l'ADN purifié présente bien un signal d'hybridation avec la sonde EPSPS d'*Arabidopsis thaliana*. Après l'électrophorèse, les fragments d'ADN sont transférés sur membrane liybond N d'Amersham selon le protocole de Southern décrit dans "Current Protocols in Molecular Biology". Le filtre est hybridé avec la sonde EPSPS d'*Arabidopsis thaliana* selon les conditions décrites au paragraphe 3 ci-dessus. Le clone plasmidique présentant un insert de 1,7kpb et hybridant avec la sonde EPSPS d'*Arabidopsis thaliana* a été préparé à plus grande échelle et l'ADN résultant de la lyse des bactéries purifié sur gradient de CsCl ainsi que décrit dans "Current Protocols in Molecular Biology". L'ADN purifié a été partiellement séquencé avec un kit Pharmacia en suivant les instructions du fournisseur et en utilisant comme amorces, les amorces universelles de M13 directes et inverses commandées chez le même fournisseur. La séquence partielle réalisée couvre environ 0,5 kpb. La séquence dérivée en acides aminés dans la région de la protéine mature (environ 50 résidus acides aminés) présente une identité de 100% avec la séquence aminée correspondante de l'EPSPS mature de maïs décrite dans le brevet américain USP 4 971 908). Ce clone correspondant à un fragment EcoRI de 1,7kpb de l'ADN de l' EPSP de la suspension cellulaire de maïs BMS a été nommé pRPA-ML-711. La séquence complète de ce clone a été réalisée sur les deux brins en utilisant le protocole du kit Pharmacia et en synthétisant des oligonucléotides complémentaitres et de direction opposée tous les 250 pb environ. La séquence complète de ce clone de 1713 pb obtenue est présentée par SEQ ID N° 1.
6. Obtention du clone pRPA-ML-715:
   L'analyse de la séquence du clone pRPA-ML-711 et en particulier la comparaison de la séquence d' acides aminés dérivés avec celle de maïs montre une extension de séquence de 92 pb en amont du codon GCG codant pour l'Alanine NH2-terminale de la partie mature de l'EPSPS de maïs (brevet américain USP 4 971 908). De même une extension de 288 pb en aval du codon AAT codant pour l'asparagine COOH-terminale de la partie mature de l'EPSPS de maïs (brevet américain USP 4 971 908) est observée. Ces deux parties pourraient correspondre, pour l'extension NH2-terminale à une portion de la séquence d'un peptide de transit pour la localisation plastidiale et pour l'extension COOH-terminale à la région 3' non traduite de l'ADNc.
   Afin d'obtenir un ADNc codant pour la partie mature de l'ADNc de l'EPSPS de maïs, telle que décrite dans l' USP 4 971 908, les opérations suivantes ont été réalisées:
   a) Elimination de la région 3' non traduite: construction de pRPA-ML-712:
      Le clone pRPA-ML-711 a été coupé par l'enzyme de restriction AseI et les extrémités résultant de cette coupure rendues franches par traitement avec le fragment de Klenow de l'ADN polymérase I selon le protocole décrit dans CPMB. Une coupure par l'enzyme de restriction SacII a ensuite été effectuée. L'ADN résultant de ces opérations a été séparé par électrophorèse sur gel d'agarose LGTA/TBE (réf. CPMB) 1 %.
      Le fragment de gel contenant l'insert "AseI-extrémités franches/SacII" de 0,4 kpb a été excisé du gel et purifié selon le protocole décrit au paragraphe 5 ci-dessus. L'ADN du clone pRPA-ML-711 a été coupé par l'enzyme de restriction HindIII située dans le polylinker du vecteur de clonage pUC19 et les extrémités résultant de cette coupure ont été rendues franches par traitement avec le fragment de Klenow de l'ADN polymérase I. Une coupure par l'enzyme de restriction SacII a ensuite été effectuée. L'ADN résultant de ces manipulations a été séparé par électrophorèse sur gel d'agarose LGTA/TBE (réf. CPMB) 0,7%.
      Le fragment de gel contenant l'insert HindIII-extrémités franches/SacII de environ 3,7kpb a été excisé du gel et purifié selon le protocole décrit au paragraphe 5 ci-dessus.
      Les deux inserts ont été ligués, et 2 µl du mélange de ligation ont servi à transformer E. *coli* DHlOB ainsi que décrit plus haut au paragraphe 5.
      On analyse le contenu en ADN plasmidique de différents clones selon la procédure décrite pour pRPA-ML-711. Un des clones plasmidique retenu contient un insert EcoRI-HindIII de 1,45 kpb environ. La séquence des extrémités terminales de ce clone révèle que l'extrémité 5' de l'insert correspond exactement à l'extrémité correspondante de pRPA-ML-711 et que l'extrémité 3' terminale présente la séquence suivante:
      La séquence soulignée correspond au codon de l'acide aminé COOH-terminal asparagine, le codon suivant correspondant au codon stop de la traduction. Les nucléotides en aval correspondent à des éléments de séquence du polylinker de pUCI9. Ce clone comprenant la séquence de pRPAML-711 jusqu'au site de terminaison de la traduction de l'EPSPS mature de maïs et suivie de séquences du polylinker de pUC 19 jusqu'au site HindIII a été nommé pRPA-ML-712.
   b) Modification de l'extrémité 5' de pRPA-ML-712: construction de pRPA-ML-715
      Le clone pRPA-ML-712 a été coupé par les enzymes de restrictions PstI et HindIII. L'ADN résultant de ces manipulations a été séparé par électrophorèse sur gel d'agarose LGTA/TBE (réf. CPMB) 0,8%. Le fragment de gel contenant l'insert PstI/EcoRI de 1,3 kpb a été excisé du gel et purifié selon le protocole décrit au paragraphe 5 ci-dessus. Cet insert a été mis en ligation en présence de quantité équimoléculaire de chacun des deux oligonucléotides partiellement complémentaires, de séquence:
      ainsi qu'en présence d'ADN du plasmide pUC19 digéré par les enzymes de restrictions BamHI et HindIII.
      Deux µl du mélange de ligation ont servi à transformer *E. coli* DHlOB ainsi que décrit plus haut au paragraphe 5. Après analyse du contenu en ADN plasmidique de différents clones selon la procédure décrite ci-dessus au paragraphe 5, un des clones présentant un insert d'environ 1,3 kpb a été conservé pour analyses ultérieures. La séquence de l'extrémité 5' terminale du clone retenu révèle que la séquence ADN dans cette région est la suivante: séquence du polylinker de pUC19 des sites EcoRI à BamHI, suivi de la séquence des oligonucléotides utilisés lors du clonage, suivi du reste de la séquence présente dans pRPAML-712. Ce clone a été nommé pRPA-ML-713. Ce clone présente un codon methionine ATG inclu dans un site NcoI en amont du codon Alanine N-terminal de l'EPSPSynthase mature. De plus, les codons alanine et glycine de l'extrémité N-terminale ont été conservées, mais modifiées sur la troisième base variable : GCGGGT initial donne GCCGGC modifié.
      Le clone pRPA-ML-713 a été coupé par l'enzyme de restriction HindIII et les extrémités de cette coupure rendues franches par traitement avec le fragment de Klenow de la ADN polymérase I. Une coupure par l'enzyme de restriction SacI a ensuite été effectuée. L'ADN résultant de ces manipulations a été séparé par électrophorèse sur gel d'agarose LGTA/TBE (réf. CPMB) 0,8%. Le fragment de gel contenant l'insert "HindIII-extrémités franches/SacI" de 1,3 kpb a été excisé du gel et purifié selon le protocole décrit au paragraphe 5 ci-dessus. Cet insert a été mis en ligation en présence d'ADN du plasmide pUC19 digéré par l'enzyme de restriction XbaI et les extrémités de cette coupure rendues franches par traitement avec le fragment de Klenow de l'ADN polymérase I. Une coupure par l'enzyme de restriction SacI a ensuite été effectuée. Deux µl du mélange de ligation ont servi à transformer *E. coli* DHlOB ainsi que décrit plus haut au paragraphe 5. Après analyse du contenu en ADN plasmidique de différents clones selon la procédure décrite ci-dessus au paragraphe 5, un des clones présentant un insert d'environ 1,3 kpb a été conservé pour analyses ultérieures. La séquence des extrémités terminales du clone retenu révèle que la séquence ADN est la suivante: séquence du polylinker de pUC19 des sites EcoRI à SacI, suivie de la séquence des oligonucléotides utilisés lors du clonage délétée des 4 pb GATCC de l'oligonucléotide 1 décrit ci-dessus, suivi du reste de la séquence présente dans pRPA-ML-712 jusqu'au site HindIII et séquence du polylinker de pUC19 de XbaI à HindIII. Ce clone a été nommé pRPA-ML-715.
7. Obtention d'un ADNc codant pour une EPSPS de maïs mutée
   Toutes les étapes de mutagénèse ont été réalisées avec le U.S.E. mutagenesis kit de Pharmacia en suivant les instructions du fournisseur. Le principe de ce système de mutagénèse est le suivant: l'ADN plasmidique est dénaturé par la chaleur et réassocié en présence d'un excès molaire d'une part de l'oligonucléotide de mutagénèse, et d'autre part d'un oligonucléotide permettant d'éliminer un site d'enzyme de restriction unique présent dans le polylinker. Après l'étape de réassociation, la synthèse du brin complémentaire est réalisée par l'action de la T4 ADN polymérase en présence de T4 ADN ligase et de protéine du gène 32 dans un tampon approprié fourni. Le produit de synthèse est incubé en présence de l'enzyme de restriction, dont le site est supposé avoir disparu par mutagénèse. La souche d'*E. coli* présentant, en particulier, la mutation mutS est utilisée comme hôte pour la transformation de cet ADN. Après croissance en milieu liquide, l'ADN plasmidique total est préparé, incubé en présence de l'enzyme de restriction utilisée précédemment. Après ces traitements, la souche d'*E*. *coli* DHlOB est utilisée comme hôte pour la transformation. L'ADN plasmidique des clones isolés est préparé et la présence de la mutation introduite vérifiée par séquençage.
   A)- modifications de sites ou de séquence sans incidence a priori sur le caractère de résistance de l'EPSPS de maïs aux produits inhibiteurs compétitifs de l'activité EPSP synthase: élimination d'un site NcoI interne de pRPA-ML-715.
      La séquence de pRPA-ML-715 est numérotée arbitrairement en plaçant la première base du codon Alanine N-terminal GCC en position 1. Cette séquence présente un site NcoI en position 1217. L'oligonucléotide de modification du site présente la séquence :
      Après séquençage selon les références données ci-dessus, la séquence lue après mutagénèse correspond à celle de l'oligonucléotide utilisé. Le site NcoI a bien été éliminé et la traduction en acides aminés dans cette région conserve la séquence initiale présente sur pRPA-ML-715.
      Ce clone a été nommé pRPA-ML-716.
      La séquence de 1340 bp de ce clone est présentée SEQ ID N° 2 et SEQ ID N° 3.
   B) modifications de séquence permettant l'augmentation du caractère de résistance de l'EPSPS de maïs aux produits inhibiteurs compétitifs de l'activité EPSP synthase.
      Les oligonucléotides suivants ont été utilisés :
      a) mutation Thr 102 à Ile.
         b) mutation Pro 106 à Ser.
         c) mutations Gly 101 à Ala et Thr 102 à Ile.
         d) mutations Thr 102 à IIe et Pro 106 à Ser.

Après séquençage, la séquence lue après mutagénèse sur les trois fragments mutés est identique à la séquence de l'ADN parental pRPA-ML-716 à l'exception de la région mutagénéisée qui correspond à celle des oligonucléotides de mutagénèse utilisés.Ces clones ont été nommés : pRPA-ML-717 pour la mutation Thr 102 à Ile, pRPA-ML-718 pour la mutation Pro 106 à Ser, pRPA-ML-719 pour les mutations Gly 101 à Ala et Thr 102 à Ile et pRPA-ML-720 pour les mutations Thr 102 à Ile et Pro 106 à Ser.

La séquence de 1340 bp de pRPA-ML-720 est présentée SEQ ID N° 4 et SEQ ID N° 5.

L'insert NcoI-HindIII de 1395 pb est à la base de toutes les constructions utilisées pour la transformation des plantes pour l'introduction de la résistance aux herbicides inhibiteurs compétitifs de l'EPSPS et en particulier la résistance au glyphosate. Cet insert sera nommé dans la suite des descriptions "le double mutant de l'EPSPS de maïs".

### Exemple 2:

Tolérance au glyphosate des différents mutants in vitro.
2.a: Extraction de l'EPSP synthase.
   Les différents gènes d'EPSP synthases sont introduits sous forme d'une cassette NcoI-HindIII dans le vecteur plasmidique pTrc99a (Pharmacia, ref : 27-5007-01) coupé par NcoI et HindIII. Les *E. coli* DH10B recombinantes surexprimant les différents EPSP synthases sont soniquées dans 40 ml de tampon par 10 g de cellules culottées et lavées avec ce même tampon (tris HCl 200 mM pH 7,8, mercaptoethanol 50 mM, EDTA 5 mM et PMSF 1 mM), auxquels on ajoute 1 g de polyvinylpirrolidone. La suspension est agitée pendant 15 minutes à 4°C, puis centrifugée 20 minutes à 27000g et 4°C.
   Le surnageant est additionné de sulfate d'ammonium pour amener la solution à 40% de la saturation en sulfate d'ammonium. Le mélange est centrifugé 20 minutes à 27000g et 4°C. Le nouveau surnageant est additionné de sulfate d'ammonium pour amener la solution à 70% de la saturation en sulfate d'ammonium. Le mélange est centrifugé 30 minutes à 27000g et 4°C. L'EPSP synthase, présente dans ce culot protéique, est reprise dans 1 ml de tampon (tris HCl 20 mM pH 7,8 et mercaptoethanol 50 mM). Cette solution est dialysée une nuit contre deux litres de ce même tampon à 4°C.
2.b: Activité enzymatique.
   L'activité de chaque enzyme ainsi que sa résistance au glyphosate est mesurée in vitro sur 10 minutes à 37°C dans le mélange réactionnel suivant: acide maléique 100 mM pH 5,6, phosphoénol pyruvate 1 mM, shikimate-3-phosphate 3 mM (préparé selon Knowles P.F. et Sprinson D.B. 1970. Methods in Enzymol 17A, 351-352 à partir de *Aerobacter aerogenes* strain ATCC 25597) et fluorure de potassium 10 mM. L'extrait enzymatique est ajouté au dernier moment après l'addition de glyphosate dont la concentration finale varie de 0 à 20 mM.
   L'activité est mesurée par dosage du phosphate libéré selon la technique de Tausky H.A. et Shorr E. 1953. J. Biol. Chem. 202, 675-685.
   Dans ces conditions, l'enzyme sauvage (WT) est inhibée à 85% dès la concentration de 0,12 mM de glyphosate. A cette concentration, l'enzyme mutante connue Ser106 n'est inhibée qu'à 50% et les trois autres mutants IIe102, Ile102/Ser106, Ala101/Ile102 ne sont pas ou peu inhibées.
   Il faut multiplier la concentration de glyphosate par dix, soit 1,2 mM, pour inhiber l'enzyme mutante Ile102 à 50%, les mutants Ile102/Ser106, Ala/Ile et Ala n'étant toujours pas inhibés.

Il faut noter que l'activité des mutants Ala/Ile et Ala n'est pas inhibée jusqu'à des concentrations de 10 mM de glyphosate, et que celle du mutant Ile102/Ser106 n'est pas réduite même si la concentration en glyphosate est multipliée par 2, soit 20 mM.

### Exemple 3:

### Résistance des plantes de tabac transformés.

1-1- Transformation.
   Le vecteur pRPA-RD-173 est introduit dans la souche d'*Agrobacterium tumefaciens* EHA101 (Hood et al.,1987) porteuse du cosmide pTVK291 (Komari et al.,1986). La technique de transformation est basée sur la procédure de Horsh et al.(1985).
1-2- Régénération.
   La régénération du tabac PBD6 (provenance SEITA France) à partir d'explants foliaires est réalisée sur un milieu de base Murashige et Skoog (MS) comprenant 30g/l de saccharose ainsi que 200 µg/ml de kanamycine. Les explants foliaires sont prélevés sur des plantes cultivées en serre ou *in vitro* et transformées selon la technique des disques foliaires (Science, 1985,Vol 227,p. 1229-1231) en trois étapes successives: la première comprend l'induction des pousses sur un milieu additionné de 30g/l de saccharose contenant 0,05 mg/l d'acide naphtylacétique (ANA) et 2mg/l de benzylaminopurine (BAP) pendant 15 jours. Les pousses formées au cours de cette étape sont ensuite développées pendant 10 jours par culture sur un milieu MS additionné de 30g/l de saccharose mais ne contenant pas d'hormone. Puis on prélève des pousses développées et on les cultive sur un milieu d'enracinement MS à teneur moitié en sels, vitamines et sucre et ne contenant pas d'hormone. Au bout d'environ 15 jours, les pousses enracinées sont passées en terre.
1-3- Résistance au glyphosate.
   Vingt plantes transformées ont été régénérées et passées en serre pour la construction pRPA-RD-173. Ces plantes ont été traitées en serre au stade 5 feuilles avec une suspension acqueuse de RoundUp correspondant à 0,8kg de matière active glyphosate par hectare.
   Les résultats correspondent à l'observation d'indices de phytotoxicité relevés 3 semaine après traitement. Dans ces conditions, on constate que les plantes transformées par la construction pRPA-RD-173 présentent une très bonne tolérance alors que les plantes témoins non transformées sont complètement détruites.
   Ces résultats montrent clairement l'amélioration apportée par l'utilisation d'un gène chimère selon l'invention pour un même gène codant pour la tolérance au glyphosate.

### Exemple 4:

### Transformation et sélection de cellules de maïs.

Des cellules de maïs BMS (Black Mexican Sweet) en phase exponentielle de croissance sont bombardées avec la contruction pRPA-RD-130 selon le principe et le protocole décrit par Klein et al 1987 (Klein TM, Wolf ED, Wu R and Sandford JC (1987): High velocity microprojectiles for delivering nucleic acids into livings cells NATURE vol 327 p 70-73)

Deux jours après le bombardement, les cellules sont transférées sur le même milieu contenant 2mM de N(phosphométhyl)glycine.

Après 8 semaines de sélection sur ce milieu, des cals se développant sont sélectionnés puis amplifiés et analysés par PCR et révèlent clairement la présence du gène chimère PTO-EPSPS.

Les cellules non bombardées et mises en croissance sur le même milieu contenant 2mM de N(phosphométhylglycine) sont bloquées par l'herbicide et ne se développent pas.

Les plantes transformées selon l'invention peuvent être utilisées comme parents pour l'obtention de lignées et d'hybrides ayant le caractère phénotypique correspondant à l'expression du gène chimère introduit.

### Description des constructions des plamides

pRPA-RD-124: Addition d'un signal de polyadénylation "nos" à pRPA-ML-720 avec création d'une cassette de clonage contenant le gène d'EPSPS double mutant de maïs (Thr 102 → Ile et Pro 106 → Ser). pRPA-ML-720 est digéré avec Hind III, traité avec le fragment de Klenow de l'ADN polymérase I d'*E*. *coli* pour produire une extrémité franche. On effectue une seconde digestion avec Nco I et le fragment EPSPS est purifié. Le gène EPSPS est ensuite ligué avec pRPA-RD-12 purifié (une cassette de clonage contenant le signal de polyadénylation de la nopaline synthase) pour donner pRPA-RD-124. Pour obtenir le vecteur pRPA-RD-12 purifié utile, il a fallu que celui-ci soit préalablement digéré par SalI, traité avec l'ADN polymérase de Klenow, puis digéré une seconde fois avec NcoI.

pRPA-RD-125: Addition d'un peptide de transit optimisé (PTO) à pRPA-RD-124 avec création d'une cassette de clonage contenant le gène d'EPSPS ciblé sur les plasmides. pRPA-RD-7 (demande de brevet européen EP 652 286) est digérée avec Sph I, traité avec la T4 ADN polymérase, puis digérée avec Spe 1 et le fragment PTO est purifié. Ce fragment PTO est cloné dans pRPA-RD-124 qui a été préalablement digérée par NcoI, traité avec l'ADN polymérase de Klenow pour enlever la partie protubérante 3', puis digérée par Spe I. Ce clone est alors séquencé pour assurer la fusion traductionnelle correcte entre le PTO et le gène d'EPSPS. On obtient alors pRPA-RD-125.

pRPA-RD-130: Addition du promoteur d'histone de maïs H3C4 et de séquences d'intron 1 adhl de pRPA-RD-123 (demande de brevet EP 507 698) à pRPA-RD-125 avec création d'une cassette pour expression dans les plantes pour l'expression du gène d'EPSPS double mutant dans les tissus de monocotylédones. pRPA-RD-123 (une cassette contenant le promoteur d'histone de maïs H3C4 fusionné avec l'intron 1 adhl) est digérée avec Nco I et Sac I. Le fragment d'ADN contenant le promoteur dérivé de pRPA-RD-123 est ensuite purifié et ligué avec pRPA-RD-125, qui a été préalablement digéré avec Nco I et Sac I.

pRPA-RD-159: Addition du promoteur double d'histone de d'*arabidopsis* H4A748 (demande de brevet EP 507 698) à pRPA-RD-125 avec création d'une cassette pour expression dans les plantes pour l'expression du gène "PTO- gène d'EPSPS double mutant" dans les tissus de dicotylédones. pR.PA-RD-132 (une cassette contenant le promoteur double H4A748 (demande de brevet EP 507 698)) est digérée avec Nco I et Sac I. Le fragment purifié du promoteur est ensuite cloné dans qui a été digéré avec Eco I et Sac I.

pRPA-R.D-173: Addition du gène "promoteur H4A748-PTO-gène d'EPSPS double mutant" de pRPA-RD-159 dans plasmide pRPA-BL-150A (demande de brevet européen 508 909) avec création d'un vecteur de transformation *Agrobacterium tumefaciens*. pRPA-RD-159 est digéré avec Not 1 et traité avec la polymérase de Klenow. Ce fragment est ensuite cloné dans pRPA-BL-150A avec Sma I.

### SEQUENCE LISTING

(i) GENERAL INFORMATION:
   (i) APPLICANT: Lebrun, Michel De Rose, Richard T Sailland, Alain
   (ii) TITLE OF INVENTION: 5-enol pyruvylshikimate-3-phosphate synthase mutee, gens codant pour cette proteine et plantes transformées contenant ce gene
   (iii) NUMBER OF SEQUENCES: 5
   (iv) CORRESPONCENCE ADDRESS:
      (A) ADDRESSEE: Francois Chretien
      (B) STREET: 1420 rue Pirre Baizet
      (C) CITY: Lyon Cedex 09
      (E) COUNTRY: France
      (F) ZIP: 69263
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release 1.0, Version 1.25
   (vi) CURRENT APPLICATION DATA:
      (A) APPLICATION NUMBER:
      (B) FILING DATE:
      (C) CLASSIFICATION:
   (viii) ATTORNEY/AGENT IMFORMATION:
      (A) NAME: Chretion, François
   (ix) TELECOMMUNICATION INFORMATION:
      (A) TELEPHONE: (33)72-29-26-46
      (B) TELEFAX: (33)72-29-28-13
(2) INFORMATION FOR SEQ ID NO:1:
   (i) SEQUENCE CHARACTERISTICS:
      (A) LENGTH: 1713 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Zea mays
      (B) SIRAIN: Black Mexican Sweet
      (E) TISSUE TYPE: Callus
   (vii) IMMEDIATE SOURCE:
      (A) LIBRARY: lambda gt10
      (B) CLONE: pRPA-ML-711
   (xi) SEQUENCE DESCRIPTION: SEQ ID NO:1:
   AATCAATTTC ACACAGGAAA CAGCTATGAC CATGATTACG AATTCGGGCC CGGGCGCGTG    60
   ATCCGGCGGC GGCAGCGGCG GCGGCGGTGC AGGCGGGTGC CGAGGAGATC GTGCTGCAGC   120
   CCATCAAGGA GATCTCCGGC ACCGTCAAGC TGCCGGGGTC CAAGTCGCTT TCCAACCGGA   180
   TCCTCCTACT CGCCGCCCTG TCCGAGGGGA CAACAGTGGT TGATAACCTG CTGAACAGTG   240
   AGGATGTCCA CTACATGCTC GGGGCCTTGA GGACTCTTGG TCTCTCTGTC GAAGCGGACA   300
   AAGCTGCCAA AAGAGCTGTA GTTGTTGGCT GTGGTGGAAA GTTCCCAGTT GAGGATGCTA   360
   AAGAGGAAGT GCAGCTCTTC TTGGGGAATG CTGGAACTGC AATGCGGCCA TTGACAGCAG   420
   CTGTTACTGC TGCTGGTGGA AATGCAACTT ACGTGCTTGA TGGAGTACCA AGAATGAGGG   480
   AGAGACCCAT TGGCGACTTG GTTGTCGGAT TGAAGCAGCT TGGTGCAGAT GTTGATTGTT    540
   TCCTTGGCAC TGACTGCCCA CCTGTTCGTG TCAATGGAAT CGGAGGGCTA CCTGGTGGCA    600
   AGGTCAAGCT GTCTGGCTCC ATCAGCAGTC AGTACTTGAG TGCCTTGCTG ATGGCTGCTC    660
   CTTTGGCTCT TGGGGATGTG GAGATTGAAA TCATTGATAA ATTAATCTCC ATTCCGTACG    720
   TCGAAATGAC ATTGAGATTG ATGGAGCGTT TTGGTGTGAA AGCAGAGCAT TCTGATAGCT    780
   GGGACAGATT CTACATTAAG GGAGGTCAAA AATACAAGTC CCCTAAAAAT GCCTATGTTG    840
   AAGGTGATGC CTCAAGCGCA AGCTATTTCT TGGCTGGTGC TGCAATTACT GGAGGGACTG    900
   TGACTGTGGA AGGTTGTGGC ACCACCAGTT TGCAGGGTGA TGTGAAGTTT GCTGAGGTAC    960
   TGGAGATGAT GGGAGCGAAG GTTACATGGA CCGAGACTAG CGTAACTGTT ACTGGCCCAC   1020
   CGCGGGAGCC ATTTGGGAGG AAACACCTCA AGGCGATTGA TGTCAACATG AACAAGATGC   1080
   CTGATGTCGC CATGACTCTT GCTGTGGTTG CCCTCTTTGC CGATGGCCCG ACAGCCATCA   1140
   GAGACGTGGC TTCCTGGAGA GTAAAGGAGA CCGAGAGGAT GGTTGCGATC CGGACGGAGC   1200
   TAACCAAGCT GGGAGCATCT GTTGAGGAAG GGCCGGACTA CTGCATCATC ACGCCGCCGG   1260
   AGAAGCTGAA CGTGACGGCG ATCGACACGT ACGACGACCA CAGGATGGCC ATGGCCTTCT   1320
   CCCTTGCCGC CTGTGCCGAG GTCCCCGTCA CCATCCGGGA CCCTGGGTGC ACCCGGAAGA   1380
   CCTTCCCCGA CTACTTCGAT GTGCTGAGCA CTTTCGTCAA GAATTAATAA AGCGTGCGAT   1440
   ACTACCACGC AGCTTGATTG AAGTGATAGG CTTGTGCTGA GGAAATACAT TTCTTTTGTT   1500
   CTGTTTTTCT CTTTCACGGG ATTAAGTTTT GAGTCTGTAA CGTTAGTTGT TTGTAGCAAG   1560
   TTTCTATTTC GGATCTTAAG TTTGTGCACT GTAAGCCAAA TTTCATTTCA AGAGTGGTTC     1620
   GTTGGAATAA TAAGAATAAT AAATTACGTT TCAGTGAAAA AAAAAAAAAA AAAAAAAAAA     1680
   AAAAAAAAAA AAAAAAAAAA AACCCGGGAA TTC                                1713
(2) IMFORMATIOM FOR SEQ ID NO:2:
   (i) SEQUENCE CHARACTERISTICS:
      (A) LENGTH: 1340 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGRNISH: Zea mays
      (B) STRAIN: Black Mexican Sweet
   (vii) IMMEDIATE SOURCE:
      (B) CLONE: pRPA-ML-716
   (ix) FEATURE:
      (A) NAME/KEY: CDS
      (B) LOCATION: 6..1337
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 444 amino acide
      (B) TYPE: amino acid
      (D) TOPOLOGY: linear
   (ii) MOLECULE TYPE: protein
   (xi) SEQUENCE DESCRIPTION: SEQ ID NO:3:
   Ala Gly Ala Glu Glu Ile Val Leu Gln Pro Ile Lys Glu Ile Ser Gly
    1               5                  10                  15
   Thr Val Lys Leu Pro Gly Ser Lys Ser Leu Ser Asn Arg Ile Leu Leu
               20                  25                  30
   Leu Ala Ala Leu Ser Glu Gly Thr Thr Val Val Asp Asn Leu Leu Asn
           35                  40                  45
   Ser Glu Asp Val His Tyr Met Leu Gly Ala Leu Arg Thr Leu Gly Leu
       50                  55                  60
   Ser Val Glu Ala Asp Lys Ala Ala Lys Arg Ala Val Val Val Gly Cys
   65                  70                  75                  80
   Gly Gly Lys Phe Pro Val Glu Asp Ala Lys Glu Glu Val Gln Leu Phe
                   85                  90                  95
   Leu Gly Asn Ala Gly Thr Ala Met Arg Pro Leu Thr Ala Ala Val Thr
               100                 105                 110
   Ala Ala Gly Gly Asn Ala Thr Tyr Val Leu Asp Gly Val Pro Arg Met
           115                 120                 125
   Arg Glu Arg Pro Ile Gly Asp Leu Val Val Gly Leu Lys Gln Leu Gly
       130                 135                 140
   Ala Asp Val Asp Cys Phe Leu Gly Thr Asp Cys Pro Pro Val Arg Val
   145                 150                 155                 160
   Asn Gly Ile Gly Gly Leu Pro Gly Gly Lys Val Lys Leu Ser Gly Ser
                   165                 170                 175
   Ile Ser Ser Gln Tyr Leu Ser Ala Leu Leu Met Ala Ala Pro Leu Ala
               180                 185                 190
   Leu Gly Asp Val Glu Ile Glu Ile Ile Asp Lys Leu Ile Ser Ile Pro
           195                 200                 205
   Tyr Val Glu Met Thr Leu Arg Leu Met Glu Arg Phe Gly Val Lys Ala
       210                 215                 220
   Glu His Ser Asp Ser Trp Asp Arg Phe Tyr Ile Lys Gly Gly Gln Lys
   225                 230                 235                 240
   Tyr Lys Ser Pro Lys Asn Ala Tyr Val Glu Gly Asp Ala Ser Ser Ala
                   245                 250                 255
   Ser Tyr Phe Leu Ala Gly Ala Ala Ile Thr Gly Gly Thr Val Thr Val
   260 265 270
   Glu Gly Cys Gly Thr Thr Ser Leu Gln Gly Asp Val Lys Phe Ala Glu
   275 280 285
   Val Leu Glu Met Met Gly Ala Lys Val Thr Trp Thr Glu Thr Ser Val
   290 295 300
   Thr Val Thr Gly Pro Pro Arg Glu Pro Phe Gly Arg Lys His Leu Lys
   305 310 315 320
   Ala Ile Asp Val Asn Met Asn Lys Met Pro Asp Val Ala Met Thr Leu
   325 330 335
   Ala Val Val Ala Leu Phe Ala Asp Gly Pro Thr Ala Ile Arg Asp Val
   340 345 350
   Ala Ser Trp Arg Val Lys Glu Thr Glu Arg Met Val Ala Ile Arg Thr
   355 360 365
   Glu Leu Thr Lys Leu Gly Ala Ser Val Glu Glu Gly Pro Asp Tyr Cys
   370 375 380
   Ile Ile Thr Pro Pro Glu Lys Leu Asn Val Thr Ala Ile Asp Thr Tyr
   385 390 395 400
   Asp Asp His Arg Met Ala Met Ala Phe Ser Leu Ala Ala Cys Ala Glu
   405 410 415
   Val Pro Val Thr Ile Arg Asp Pro Gly Cys Thr Arg Lys Thr Phe Pro
   420 425 430
   Asp Tyr Phe Asp Val Leu Ser Thr Phe Val Lys Asn
   435 440
(2) INFORMATION FOR SEQ ID NO:4:
   (i) SEQUENCE CHARACTERISTICS:
      (A) LENGTH: 1340 base pairs
      (B) TYPE: nucleic acid
      (C) STRANDEDNESS: double
      (D) TOPOLOGY: linear
   (ii) MOLECULE TYPE: cDNA
   (vi) ORIGINAL SOURCE:
      (A) ORGANISM: Zea maya
      (B) STRAIN: Black Mexican Sweet
   (vii) IMMEDIATE SOURCE:
      (B) CLONE: pRPA-ML-720
   (ix) FEATURE:
      (A) NAME/KEY: CDS
      (B) LOCATION: 6..1337
   (xi) SEQUENCE DESCRIPTION: SEQ ID NO:4:
   CCATG GCC GGC GCC GAG GAG ATC GTG CTG CAG CCC ATC AAG GAG ATC       47
         Ala Gly Ala Glu Glu Ile Val Leu Gln Pro Ile Lys Glu Ile
          1               5                  10
   TCC GGC ACC GTC AAG CTG CCG GGG TCC AAG TCG CTT TCC AAC CGG ATC     95
   Ser Gly Thr Val Lys Leu Pro Gly Ser Lys Ser Leu Ser Asn Arg Ile
    15                  20                  25                  30
   CTC CTA CTC GCC GCC CTG TCC GAG GGG ACA ACA GTG GTT GAT AAC CTG    143
   Leu Leu Leu Ala Ala Leu Ser Glu Gly Thr Thr Val Val Asp Asn Leu
                    35                  40                  45
   CTG AAC AGT GAG GAT GTC CAC TAC ATG CTC GGG GCC TTG AGG ACT CTT    191
   Leu Asn Ser Glu Asp Val His Tyr Met Leu Gly Ala Leu Arg Thr Leu
                50                  55                  60
   GGT CTC TCT GTC GAA GCG GAC AAA GCT GCC AAA AGA GCT GTA GTT GTT    239
   Gly Leu Ser Val Glu Ala Asp Lys Ala Ala Lys Arg Ala Val Val Val
            65                  70                  75
   GGC TGT GGT GGA AAG TTC CCA GTT GAG GAT GCT AAA GAG GAA GTG CAG      287
   Gly Cys Gly Gly Lys Phe Pro Val Glu Asp Ala Lys Glu Glu Val Gln
        80                  85                  90
   CTC TTC TTG GGG AAT GCT GGA ATC GCA ATG CGG TCC TTG ACA GCA GCT      335
   Leu Phe Leu Gly Asn Ala Gly Ile Ala Met Arg Ser Leu Thr Ala Ala
    95                 100                 105                 110
   GTT ACT GCT GCT GGT GGA AAT GCA ACT TAC GTG CTT GAT GGA GTA CCA      383
   Val Thr Ala Ala Gly Gly Asn Ala Thr Tyr Val Leu Asp Gly Val Pro
                   115                 120                 125
   AGA ATG AGG GAG AGA CCC ATT GGC GAC TTG GTT GTC GGA TTG AAG CAG      431
   Arg Met Arg Glu Arg Pro Ile Gly Asp Leu Val Val Gly Leu Lys Gln
               130                 135                 140
   CTT GGT GCA GAT GTT GAT TGT TTC CTT GGC ACT GAC TGC CCA CCT GTT      479
   Leu Gly Ala Asp Val Asp Cys Phe Leu Gly Thr Asp Cys Pro Pro Val
           145                 150                 155
   CGT GTC AAT GGA ATC GGA GGG CTA CCT GGT GGC AAG GTC AAG CTG TCT      527
   Arg Val Asn Gly Ile Gly Gly Leu Pro Gly Gly Lys Val Lys Leu Ser
       160                 165                 170
   GGC TCC ATC AGC AGT CAG TAC TTG AGT GCC TTG CTG ATG GCT GCT CCT      575
   Gly Ser Ile Ser Ser Gln Tyr Leu Ser Ala Leu Leu Met Ala Ala Pro
   175                 180                 185                 190
   TTG GCT CTT GGG GAT GTG GAG ATT GAA ATC ATT GAT AAA TTA ATC TCC      623
   Leu Ala Leu Gly Asp Val Glu Ile Glu Ile Ile Asp Lys Leu Ile Ser
                   195                 200                 205
   ATT CCG TAC GTC GAA ATG ACA TTG AGA TTG ATG GAG CGT TTT GGT GTG      671
   Ile Pro Tyr Val Glu Met Thr Leu Arg Leu Met Glu Arg Phe Gly Val
               210                 215                 220
   AAA GCA GAG CAT TCT GAT AGC TGG GAC AGA TTC TAC ATT AAG GGA GGT      719
   Lys Ala Glu His Ser Asp Ser Trp Asp Arg Phe Tyr Ile Lys Gly Gly
           225                 230                 235
   CAA AAA TAC AAG TCC CCT AAA AAT GCC TAT GTT GAA GGT GAT GCC TCA      767
   Gln Lys Tyr Lys Ser Pro Lys Asn Ala Tyr Val Glu Gly Asp Ala Ser
       240                 245                 250
   AGC GCA AGC TAT TTC TTG GCT GGT GCT GCA ATT ACT GGA GGG ACT GTG      815
   Ser Ala Ser Tyr Phe Leu Ala Gly Ala Ala Ile Thr Gly Gly Thr Val
   255                 260                 265                 270
   ACT GTG GAA GGT TGT GGC ACC ACC AGT TTG CAG GGT GAT GTG AAG TTT      863
   Thr Val Glu Gly Cys Gly Thr Thr Ser Leu Gln Gly Asp Val Lys Phe
                   275                 280                 285
   GCT GAG GTA CTG GAG ATG ATG GGA GCG AAG GTT ACA TGG ACC GAG ACT      911
   Ala Glu Val Leu Glu Met Met Gly Ala Lys Val Thr Trp Thr Glu Thr
               290                 295                 300
   AGC GTA ACT GTT ACT GGC CCA CCG CGG GAG CCA TTT GGG AGG AAA CAC      959
   Ser Val Thr Val Thr Gly Pro Pro Arg Glu Pro Phe Gly Arg Lys His
           305                 310                 315
   CTC AAG GCG ATT GAT GTC AAC ATG AAC AAG ATG CCT GAT GTC GCC ATG     1007
   Leu Lys Ala Ile Asp Val Asn Met Asn Lys Met Pro Asp Val Ala Met
       320                 325                 330
   ACT CTT GCT GTG GTT GCC CTC TTT GCC GAT GGC CCG ACA GCC ATC AGA     1055
   Thr Leu Ala Val Val Ala Leu Phe Ala Asp Gly Pro Thr Ala Ile Arg
   335                 340                 345                 350
   GAC GTG GCT TCC TGG AGA GTA AAG GAG ACC GAG AGG ATG GTT GCG ATC     1103
   Asp Val Ala Ser Trp Arg Val Lys Glu Thr Glu Arg Met Val Ala Ile
                   355                 360                 365
   CGG ACG GAG CTA ACC AAG CTG GGA GCA TCT GTT GAG GAA GGG CCG GAC     1151
   Arg Thr Glu Leu Thr Lys Leu Gly Ala Ser Val Glu Glu Gly Pro Asp
               370                 375                 380
   TAC TGC ATC ATC ACG CCG CCG GAG AAG CTG AAC GTG ACG GCG ATC GAC     1199
   Tyr Cys Ile Ile Thr Pro Pro Glu Lys Leu Asn Val Thr Ala Ile Asp
           385                 390                 395
   ACG TAC GAC GAC CAC AGG ATG GCG ATG GCC TTC TCC CTT GCC GCC TGT        1247
   Thr Tyr Asp Asp His Arg Met Ala Met Ala Phe Ser Leu Ala Ala Cys
       400                 405                 410
   GCC GAG GTC CCC GTC ACC ATC CGG GAC CCT GGG TGC ACC CGG AAG ACC        1295
   Ala Glu Val Pro Val Thr Ile Arg Asp Pro Gly Cys Thr Arg Lys Thr
   415                 420                 425                 430
   TTC CCC GAC TAC TTC GAT GTG CTG AGC ACT TTC GTC AAG AAT                1337
   Phe Pro Asp Tyr Phe Asp Val Leu Ser Thr Phe Val Lys Asn
                   435                 440
   TAA                                                                    1340
(2) INFORMATION FOR SEQ ID NO:5:
   (i) SEQUENCE CHARACTERISTICS:
      (A) LENGTH: 444 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Ala Gly Ala Glu Glu Ile Val Leu Gln Pro Ile Lys Glu Ile Ser Gly
   1               5                   10                  15
   Thr Val Lys Leu Pro Gly Ser Lys Ser Leu Ser Asn Arg Ile Leu Leu
               20                  25                  30
   Leu Ala Ala Leu Ser Glu Gly Thr Thr Val Val Asp Asn Leu Leu Asn
           35                  40                  45
   Ser Glu Asp Val His Tyr Met Leu Gly Ala Leu Arg Thr Leu Gly Leu
       50                  55                  60
   Ser Val Glu Ala Asp Lys Ala Ala Lys Arg Ala Val Val Val Gly Cys
   65                  70                  75                  80
   Gly Gly Lys Phe Pro Val Glu Asp Ala Lys Glu Glu Val Gln Leu Phe
                   85                  90                  95
   Leu Gly Asn Ala Gly Ile Ala Met Arg Ser Leu Thr Ala Ala Val Thr
               100                 105                 110
   Ala Ala Gly Gly Asn Ala Thr Tyr Val Leu Asp Gly Val Pro Arg Met
           115                 120                 125
   Arg Glu Arg Pro Ile Gly Asp Leu Val Val Gly Leu Lys Gln Leu Gly
       130                 135                 140
   Ala Asp Val Asp Cys Phe Leu Gly Thr Asp Cys Pro Pro Val Arg Val
   145                 150                 155                 160
   Asn Gly Ile Gly Gly Leu Pro Gly Gly Lys Val Lys Leu Ser Gly Ser
                   165                 170                 175
   Ile Ser Ser Gln Tyr Leu Ser Ala Leu Leu Met Ala Ala Pro Leu Ala
               180                 185                 190
   Leu Gly Asp Val Glu Ile Glu Ile Ile Asp Lys Leu Ile Ser Ile Pro
           195                 200                 205
   Tyr Val Glu Met Thr Leu Arg Leu Met Glu Arg Phe Gly Val Lys Ala
       210                 215                 220
   Glu His Ser Asp Ser Trp Asp Arg Phe Tyr Ile Lys Gly Gly Gln Lys
   225                 230                 235                 240
   Tyr Lys Ser Pro Lys Asn Ala Tyr Val Glu Gly Asp Ala Ser Ser Ala
                   245                 250                 255
   Ser Tyr Phe Leu Ala Gly Ala Ala Ile Thr Gly Gly Thr Val Thr Val
               260                 265                 270
   Glu Gly Cys Gly Thr Thr Ser Leu Gln Gly Asp Val Lys Phe Ala Glu
           275                 280                 285
   Val Leu Glu Met Met Gly Ala Lys Val Thr Trp Thr Glu Thr Ser Val
   290 295 300
   Thr Val Thr Gly Pro Pro Arg Glu Pro Phe Gly Arg Lys His Leu Lys
   305 310 315 320
   Ala Ile Asp Val Asn Met Asn Lys Met Pro Asp Val Ala Met Thr Leu
   325 330 335
   Ala Val Val Ala Leu Phe Ala Asp Gly Pro Thr Ala Ile Arg Asp Val
   340 345 350
   Ala Ser Trp Arg Val Lys Glu Thr Glu Arg Met Val Ala Ile Arg Thr
   355 360 365
   Glu Leu Thr Lys Leu Gly Ala Ser Val Glu Glu Gly Pro Asp Tyr Cys
   370 375 380
   Ile Ile Thr Pro Pro Glu Lys Leu Asn Val Thr Ala Ile Asp Thr Tyr
   385 390 395 400
   Asp Asp His Arg Met Ala Met Ala Phe Ser Leu Ala Ala Cys Ala Glu
   405 410 415
   Val Pro Val Thr Ile Arg Asp Pro Gly Cys Thr Arg Lys Thr Phe Pro
   420 425 430
   Asp Tyr Phe Asp Val Leu Ser Thr Phe Val Lys Asn
   435 440

## Revendications

1. Plante transformée ayant une tolérance accrue aux herbicides de la famille des phosphométhylglycines laquelle plante est obtenue par régénration de cellules transformées contenant un gène chimère lequel comprend, dans le sens de la transcription :
une zone promotrice composée d'au moins un promoteur ou fragment de promoteur de gène s'exprimant naturellement dans les plantes,
une zone peptide de transit permettant le relargage d'une protéine mature dans le compartiment plastidial,
une séquence d'un gène codant pour une enzyme de tolérance au glyphosate, et
une zone signal de terminaison de la transcription non traduite en 3',
**caractérisée en ce que** l'enzyme de tolérance au glyphosate est une 5-enol pyruvylshikimate-3-phosphate synthase (EPSPS) d'origine végétale mutée comprenant au moins une substitution Thréonine 102 par l'Isoleucine et une substitution Proline 106 par la Sérine.

2. Plante selon la revendication 1, **caractérisée en ce que** l'EPSPS est d'origine de maïs.

3. Plante selon la revendication 2, **caractérisée en ce que** l'EPSPS comprend la séquence peptidique représentée par la SEQ ID NO 5.

4. Plante selon la revendication 3, **caractérisée en ce que** la séquence d'un gène codant pour une enzyme de tolérance au glyphosate comprend la partie codante de la séquence d'ADN représentée par la SEQ ID NO 4.

5. Plante selon l'une des revendications 1 à 4, **caractérisée en ce que** la zone promotrice comprend un promoteur de virus de plante.

6. Plante selon l'une des revendications 1 à 5, **caractérisée en ce que** la zone promotrice comprend un promoteur de plante.

7. Plante selon l'une des revendications 1 à 6, **caractérisée en ce que** la zone promotrice comprend un fragment d'un promoteur de gène s'exprimant naturellement dans les plantes.

8. Plante selon la revendication 7, **caractérisée en ce que** le fragment d'un promoteur s'exprimant naturellement dans les plantes est un intron actine.

9. Plante selon l'une des revendications 1 à 8, **caractérisée en ce que** la zone peptide de transit comprend une ou plusieurs unités peptides de transit.

10. Plante selon l'une des revendications 1 à 9, **caractérisée en ce que** la zone signal de terminaison de la transcription non traduite en 3' est choisie parmi celle du gène de la nopaline synthase, ou celle du gène histone H4A748 d' Arabidopsis thaliana.

11. Procédé pour la production de plantes de tolérance améliorée à un herbicide ayant pour cible l'EPSP synthase, **caractérisé en ce qu'**on transforme des cellules végétales ou protoplates avec un gène défini selon l'une des revendications 1 à 10 et qu'on soumet les cellules transformées à une régénération.

12. Plante ayant une tolérance accrue aux herbicides de la famille des phosphométhylglycines **caractérisée en ce qu'**elle est issue de croisement utilisant les plantes transformées selon l'une des revendications 1 à 10 ou obtenues par le procédé selon la revendication 11, laquelle plante est constituée de cellules comprenant un gène chimère lequel comprend, dans le sens de la transcription :
une zone promotrice composée d'au moins un promoteur ou fragment de promoteur de gène s'exprimant naturellement dans les plantes,
une zone peptide de transit permettant le relargage d'une protéine mature dans le compartiment plastidial,
une séquence d'un gène codant pour une enzyme de tolérance au glyphosate, et
une zone signal de terminaison de la transcription non traduite en 3',
**caractérisée en ce que** l'enzyme de tolérance au glyphosate est une 5-enol pyruvylshikimate-3-phosphate synthase (EPSPS) d'origine végétale mutée comprenant au moins une substitution Thréonine 102 par l'Isoleucine et une substitution Proline 106 par la Sérine.

13. Procédé de traitement des plantes avec un herbicide ayant l'EPSPS pour cible, **caractérisé en ce qu'**on applique l'herbicide à des plantes selon l'une des revendications 1 à 10 ou 12 ou obtenues par le procédé selon la revendication 11.

14. Procédé selon la revendication 13, **caractérisé en ce que** l'herbicide est le glyphosate ou un précurseur du glyphosate.

## Patentansprüche

1. Transformierte Pflanze mit einer verbesserten Toleranz gegen Herbizide der Phosphormethylglycinfamilie, wobei man diese Pflanze durch Regeneration von transformierten Zellen, die ein chimäres Gen, das in Transkriptionsrichtung eine Promotorregion, die sich aus mindestens einem Promotor oder Promotorfragment eines Gens, das auf natürliche Weise in den Pflanzen exprimiert wird, zusammensetzt,
eine Leitpeptidregion, die das Targeting eines reifen Proteins in das plastidäre Kompartiment gestattet,
eine Sequenz eines Gens, das für ein Glyphosate-Toleranzenzym codiert, sowie
eine 3'-untranslatierte Transkriptionsterminationssignalregion,
umfaßt, enthalten, erhält, **dadurch gekennzeichnet, daß** es sich bei dem Glyphosate-Toleranzenzym um eine mutierte 5-Enolpyruvylshikimat-3-phosphat-Synthase (EPSPS) pflanzlichen Ursprungs, die mindestens eine Substitution von Threonin 102 durch Isoleucin und eine Substitution von Prolin 106 durch Serin umfaßt.

2. Pflanze nach Anspruch 1, **dadurch gekennzeichnet, daß** die EPSPS aus Mais stammt.

3. Pflanze nach Anspruch 2, **dadurch gekennzeichnet, daß** die EPSPS die durch SEQ ID Nr. 5 dargestellte Peptidsequenz umfaßt.

4. Pflanze nach Anspruch 3, **dadurch gekennzeichnet, daß** die Sequenz eines Gens, das für ein Glyphosate-Toleranzenzym codiert, den Codierabschnitt der durch SEQ ID Nr. 4 dargestellten DNA-Sequenz umfaßt.

5. Pflanze nach einem der Ansprüche 1 bis 4, **dadurch gekennzeichnet, daß** die Promotorregion einen Pflanzenviruspromotor umfaßt.

6. Pflanze nach einem der Ansprüche 1 bis 5, **dadurch gekennzeichnet, daß** die Promotorregion einen Pflanzenpromotor umfaßt.

7. Pflanze nach einem der Ansprüche 1 bis 6, **dadurch gekennzeichnet, daß** die Promotorregion ein Fragment eines Promotors eines Gens, das natürlicherweise in den Pflanzen exprimiert wird, umfaßt.

8. Pflanze nach Anspruch 7, **dadurch gekennzeichnet, daß** es sich bei dem Fragment eines Promotors, der natürlich in diesen Pflanzen exprimiert wird, um ein Aktin-Intron handelt.

9. Pflanze nach einem der Ansprüche 1 bis 8, **dadurch gekennzeichnet, daß** die Leitpeptidregion eine oder mehrere Leitpeptideinheiten umfaßt.

10. Pflanze nach einem der Ansprüche 1 bis 9, **dadurch gekennzeichnet, daß** die 3'-untranslatierte Transkriptionsterminationssignalregion aus der des Nopalinsynthasegens oder der des Histon-Gens H4A748 von Arabidopsis thaliana gewählt wird.

11. Verfahren zur Herstellung von Pflanzen mit verbesserter Toleranz gegen ein Herbizid, der die EPSP-Synthase als Angriffsort dient, **dadurch gekennzeichnet, daß** man Pflanzenzellen oder - protoplasten mit einem definierten Gen nach einem der Ansprüche 1 bis 10 transformiert und die transformierten Zellen regeneriert.

12. Pflanze mit einer verbesserten Toleranz gegen Herbizide der Phosphomethylglyceninfamilie, **dadurch gekennzeichnet, daß** sie aus einer Kreuzung, bei der die transformierten Pflanzen nach einem der Ansprüche 1 bis 10 oder die nach dem Verfahren nach Anspruch 11 erhaltenen Pflanzen verwendet werden, hervorgeht, wobei sich die Pflanze aus Zellen zusammensetzt, die ein chimäres Gen, das in Transkriptionsrichtung
eine Promotorregion, die sich aus mindestens einem Promotor oder Promotorfragment eines Gens, das auf natürliche Weise in den Pflanzen exprimiert wird, zusammensetzt,
eine Leitpeptidregion, die das Targeting eines reifen Proteins in das plastidäre Kompartiment gestattet,
eine Sequenz eines Gens, das für ein Glyphosate-Toleranzenzym codiert, sowie
eine 3'-untranslatierte Transkriptionsterminationssignalregion,
umfaßt, umfassen, **dadurch gekennzeichnet, daß** es sich bei dem Glyphosate-Toleranzenzym um eine mutierte 5-Enolpyrovylshikimat-3-phosphat-Synthase (EPSPS) pflanzlichen Ursprungs, die mindestens eine Substitution von Threonin 102 durch Isoleucin und eine Substitution von Prolin 106 durch Serin umfaßt.

13. Verfahren zur Behandlung von Pflanzen mit einem Herbizid, dem die EPSPS als Angriffspunkt dient, **dadurch gekennzeichnet, daß** man das Herbizid auf Pflanzen nach einem der Ansprüche 1 bis 10 oder 12 oder Pflanzen, die nach dem Verfahren nach Anspruch 11 erhalten wurden, ausbringt.

14. Verfahren nach Anspruch 13, **dadurch gekennzeichnet, daß** es sich bei dem Herbizid um Glyphosate oder eine Glyphosate-Vorstufe handelt.

## Claims

1. Transformed plant with improved tolerance to phosphomethylglycine family herbicides, which plant is obtained by regeneration of transformed cells containing a chimeric gene which comprises, in the direction of transcription,
a promoter region composed of at least one promoter or promoter fragment of a gene which is expressed naturally in plants,
a transit peptide region which allows the targeting of a mature protein into the plastidic compartment,
a sequence of a gene coding for a glyphosate tolerance enzyme, and
a 3'-untranslated transcription termination signal region,
**characterized in that** the glyphosate tolerance enzyme is a mutated 5-enolpyruvyl-shikimate-3-phosphate synthase (EPSPS) which originates from plants and comprises at least one substitution of Threonine 102 by isoleucine and one substitution of Proline 106 by serine.

2. Plant according to Claim 1, **characterized in that** the EPSPS originates from maize.

3. Plant according to Claim 2, **characterized in that** the EPSPS comprises the peptide sequence shown in SEQ ID NO 5.

4. Plant according to Claim 3, **characterized in that** the sequence of a gene which codes for a glyphosate tolerance enzyme comprises the coding segment of the DNA sequence shown in SEQ ID NO 4.

5. Plant according to any of Claims 1 to 4, **characterized in that** the promoter region comprises a plant virus promoter.

6. Plant according to any of Claims 1 to 5, **characterized in that** the promoter region comprises a plant promoter.

7. Plant according to any of Claims 1 to 6, **characterized in that** the promoter region comprises a fragment of a promoter of a gene which is expressed naturally in plants.

8. Plant according to Claim 7, **characterized in that** the fragment of a promoter which is expressed naturally in plants is an actin intron.

9. Plant according to any of Claims 1 to 8, **characterized in that** the transit peptide region comprises one or more transit peptide units.

10. Plant according to any of Claims 1 to 9, **characterized in that** the 3'-untranslated transcription termination signal region is selected from that of the nopalin synthase gene or that of the Arabidopsis thaliana histone gene H4A748.

11. Method of producing plants with an improved tolerance to a herbicide which has EPSP synthase as its target, **characterized in that** plant cells or protoplasts are transformed with a gene defined as in any of Claims 1 to 10 and **in that** the transformed cells are regenerated.

12. Plant with improved tolerance to phosphomethylglycine family herbicides, **characterized in that** it is obtained from crossing using the transformed plants according to any of Claims 1 to 10 or plants obtained by the method of Claim 11, which plant is composed of cells comprising a chimeric gene which comprises, in the direction of transcription,
a promoter region composed of at least one promoter or promoter fragment of a gene which is expressed naturally in plants,
a transit peptide region which allows the targeting of a mature protein into the plastidic compartment,
a sequence of a gene coding for a glyphosate tolerance enzyme, and
a 3'-untranslated transcription termination signal region,
**characterized in that** the glyphosate tolerance enzyme is a mutated 5-enolpyruvyl-shikimate-3-phosphate synthase (EPSPS) which originates from plants and comprises at least one substitution of Threonine 102 by isoleucine and one substitution of Proline 106 by serine.

13. Method of treating plants with a herbicide which targets EPSPS, **characterized in that** the herbicide is applied to plants according to any of Claims 1 to 10 or 12 or plants obtained by the method of Claim 11.

14. Method according to Claim 13, **characterized in that** the herbicide is glyphosate or a glyphosate precursor.
